# EUROPEAN PATENT APPLICATION

(11) **EP 3 466 309 A1**
(43) Date of publication of application: **10.04.2019**
(21) Application number: 17802250.5
(22) Date of filing: 19.05.2017
(51) Int. Cl.: A47K 3/30, A61M 35/00

(54) **DISINFECTION CUBICLE FOR PERSONAL HYGIENE AND KILLING PATHOGENIC BACTERIA AND VIRUSES**

(30) Priority: 27.05.2016 ES 201600429
(71) Applicant: Alonso Holgado, Antonio Luis, 28009 Madrid (ES)
(72) Inventor: Alonso Holgado, Antonio Luis, 28009 Madrid (ES)
(74) Representative: Álvarez López, Sonia
(86) International application number: PCT/ES2017/070334
(87) International publication number: WO 2017/203077

(57) **Abstract**

The invention relates to a disinfection cubicle (100) for personal hygiene and killing pathogenic bacteria and viruses, which comprises a cylindrical body (42) having an upper cover (41) provided with a central hole and access doors (43, 44). The cubicle (100) contains devices (3, 18, 27) that carry products for disinfection and/or personal hygiene, and also comprises: a tank (12) for accumulating compressed air for propelling said products; tubes (16, 17, 37, 38) for carrying the products to sprayers (49) disposed along the inside of the cubicle (100); a programmer (36) interposed between the tubes (16, 17, 37, 38) and the sprayers (49), for regulating propulsion; and a compressed-air pump (1) for accumulating air pressure in the tank (12). A height-adjustable seat (51) is located inside the cubicle (100).

## Description

### OBJECT OF THE INVENTION

The invention relates to a disinfection cubicle for personal hygiene and killing pathogenic bacteria and viruses, and is included within the technical sector of the disinfection of pathogens arising from contagion in elements and overalls employed for protection against viruses and pathogenic bacteria; and within the technical sector of personal hygiene and skin care with gels, rinsing and air-drying, and the application of cosmetic products.

### BACKGROUND OF THE INVENTION

Currently, both the disinfection of viruses and pathogenic bacteria and certain personal hygiene operations are performed manually. Furthermore, with the overalls employed for protection against viruses and pathogenic bacteria, their removal is necessarily a manual operation, being necessary to follow a protocol which requires a lengthy period of time; regarding certain personal hygiene operations, such as that of the elderly , currently this is likewise performed manually, including the application of gels and cosmetic products; their access to bathing facilities for the performance of this personal hygiene also entailing complications.

The applicant is not aware of any mobile, independent cubicle capable of performing the programmed consecutive operations performed by the cubicle of the invention.

### DESCRIPTION OF THE INVENTION

The purpose of the disinfection cubicle for personal hygiene and killing pathogenic bacteria and viruses is the application of products intended for the elimination of viruses or bacteria on biological protection overalls or elements of the type used for protection against contagion, and also for use in the application of disinfectant products - either natural or chemical - for the elimination of viruses and pathogenic bacteria. A complementary or alternative use thereof is for personal hygiene, in the application of gels and cosmetic products, for example in the elderly and/or dependent persons. In fact, the cubicle of the invention is apt for both uses, as it applies products - disinfectants or hygiene products - by means of sprayers (jets/ sprinklers) located in the interior thereof.

According to the invention, the cubicle comprises a cylindrical body having an upper cover provided with a central hole for the hood of a biological protection overall against viruses and pathogenic bacteria worn by the user - or the head of the person using said cubicle for personal hygiene - to emerge, and a number of access doors. Furthermore, a height-adjustable seat is disposed in its interior.

The cubicle also comprises a number of tanks for holding disinfectant products or products for the performance of personal hygiene processes, and further comprises a tank for the accumulation of compressed air for propelling said products, and a compressed air pump for the accumulation of air pressure within the tank, a number of tubes for carrying the products to a number of sprayers disposed on the interior of the doors of the cubicle, and a programmer interposed between said tubes and said sprayers in order to systematically regulate said propulsion; that is, to perform the application of the products in a programmed manner.

The tanks comprise a first tank for detergent, cosmetic and perfumed product for skin care, a second tank for disinfectant, and a third tank for rinsing agent, each of these having a cover, a flowmeter for the product, a pressure gauge to measure the pressure and a safety valve.

The user is disposed within the cubicle and is sprayed sequentially with the various disinfectants or personal hygiene products.

In the present document the following terminology is employed:
FLOWMETER: Instrument which regulates the ingress and outflow of product.
PROGRAMMER: Instrument which regulates the distribution and time of application of a product.
SAFETY VALVE: Valve which automatically opens or closes the passage of a liquid product.
DAMPER: Mechanical element which regulates and contributes to a movement.

### BRIEF DESCRIPTION OF THE DRAWINGS

- Figure 1: portrays a view of the right-hand side of the cubicle, when facing the same;
- Figure 2: portrays a view of the left-hand side of the cubicle, when facing the same;
- Figure 3: portrays a generally frontal view of the cubicle;
- Figure 4: portrays a view similar to that portrayed in figure 3, wherein the seat, the cover and the sprayers have been eliminated;
- Figure 5: portrays a view similar to that portrayed in figure 4;
- Figure 6: portrays a view similar to that portrayed in figure 3, wherein the seat and the sprayers have been eliminated;
- Figure 7: portrays a view of the cover of the cubicle;
- Figure 8: portrays a view of the interior of the righthand door;
- Figure 9: portrays a view of the interior of the lefthand door;
- Figure 10: portrays a detail of the doors with the sprayers and safety locks.

### DESCRIPTION OF A PREFERRED EMBODIMENT OF THE INVENTION

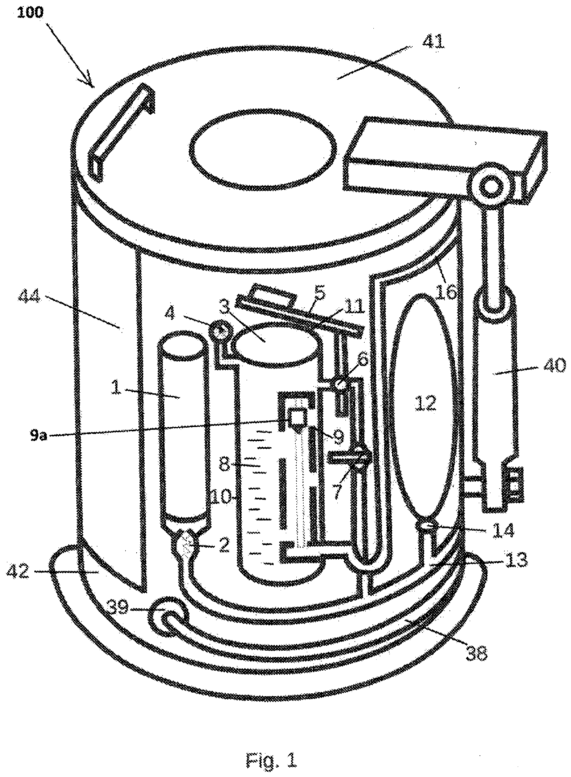

Figure 1: in this view of the right-hand side of the cubicle, when facing the same, it is seen that the body (42) of the cubicle (100) carries: an air pump (1) which provides compressed air to a tank (12) which propels the products employed in the operation of the cubicle (100), a non-return air valve (2), the aforementioned tank (12) to contain the compressed air, a first cistern (3) containing a detergent, cosmetic and perfumed product (8) for skin care, for washing the elements, overalls or persons, not portrayed, a first pressure gauge (4), a first cover (5) and a first safety valve (6) for said first cistern (3), which also has a shutoff valve (7) for the compressed air. Said figure also portrays the first flowmeter (9) of this first cistern (3) with its flowmeter body (10) and its first automatic valve (9a) which closes when the detergent is exhausted. The first cistern (3) comprises a first hinge (11) for the first cover (5). This figure also portrays the compressed air supply duct (13), a hose (14) for linking the same to the tank (12), and how said air supply duct (13) is connected to the first cistern (3) via the shutoff valve (7) and the first safety valve (6). A first connecting pipe (16) it is also seen between the first cistern and the programmer, another outlet pipe (38) transporting the products to the interior of the cubicle (100) to supply a number of sprayers (49) located on the doors (43, 44) - not portrayed in this figure for greater clarity-entering via a window (39). At the rear, the cubicle comprises a damper (40) which facilitates the opening of the cover (41) and at its front right-hand area it comprises the right-hand access door (44). The first safety valve (6) is operated (closed) by the opening movement of the first cover (5) of the first cistern (3).

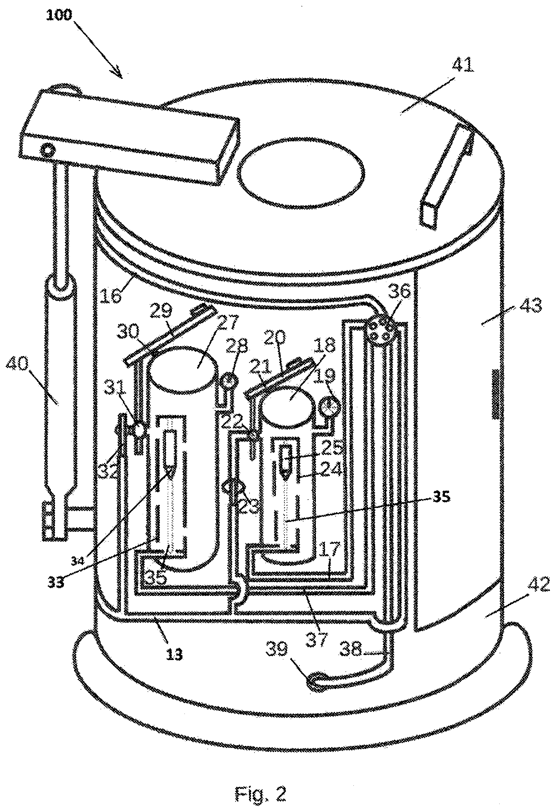

Figure 2: in this view of the left-hand side of the cubicle, when facing the same, it is seen that the body (42) of the cubicle (100) carries the compressed air supply duct (13), which is connected to the second cistern (18) of disinfectant via a second shutoff valve (23) and a second safety valve (22), and to the third cistern (27) of rinsing agent via a third shutoff valve (32) and a third safety valve (31), while the second flowmeter (24) of the second cistern (18) of disinfectant is connected to the programmer (36) by a second pipe (17); and the third flowmeter (33) of the third cistern (27) is connected to the programmer (36) by a third pipe (37). The second flowmeter (24) comprises a second automatic valve (25) which closes when the product contained therein is exhausted, and the third flowmeter (33) of the third cistern (27) comprises a third automatic valve (34) which closes when the rinsing agent is exhausted. The second cistern (18) comprises a second cover (20) and a second pressure gauge (19), and the third cistern (27) comprises a third cover (29) and a third pressure gauge (28). The second cover (20) is likewise connected to the second cistern (18) by a second hinge (21) and the third cover (29) is connected to the third cistern (27) by a third hinge (30). As may be seen in this figure and in figure 1, the valves (9a, 25, 34) comprise a number of guides (35). The output of the programmer (36) is transported by the outlet pipe (38) which likewise enters into the cubicle (100) to supply the sprayers (49) -not portrayed in this figure- located on the doors (43, 44), entering via a window (39). The second safety valve (22) is operated (closed) by the opening movement of the second cover (20) corresponding to the second cistern (18), and the third safety valve (31) is operated (closed) by the opening movement of the third cover (29) corresponding to the third cistern (27).

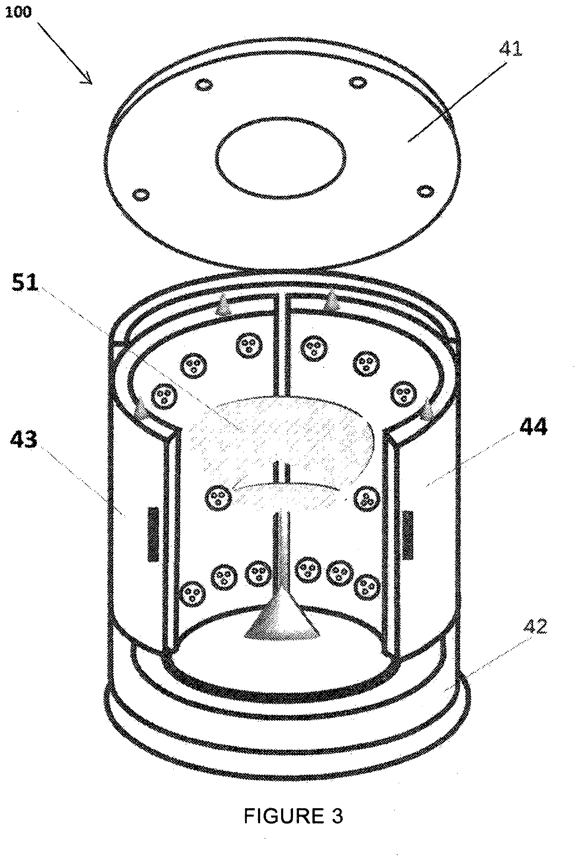

Figure 3: in this generally frontal view of the cubicle (100) it is seen that the same comprises, when facing the same, a right-hand door (44), a left-hand door (43), a height-adjustable seat (51), and the cover (41) and the body (42) of the cubicle (100).

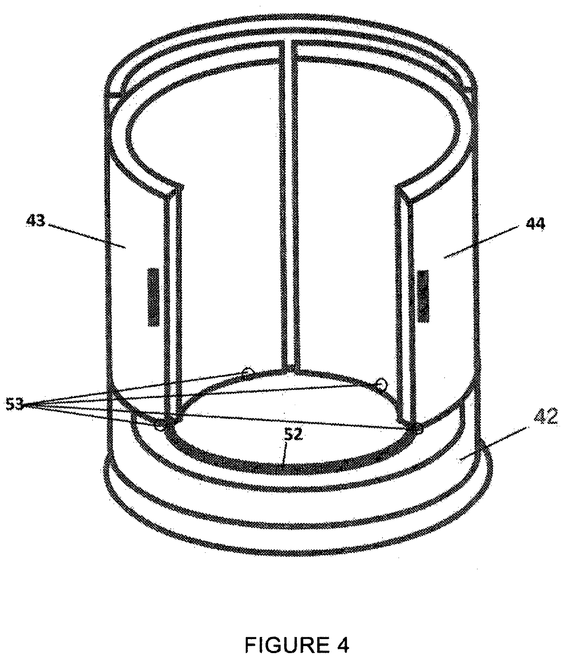

Figure 4: in this view similar to that portrayed in figure 3, wherein the seat (51), the cover (41) and the sprayers (49) have been eliminated, the right-hand door (44), the left-hand door (43), a rail (52) whereon said doors (43, 44) travel by means of roller bearings (53), and also the body (42) of the cubicle (100) are seen.

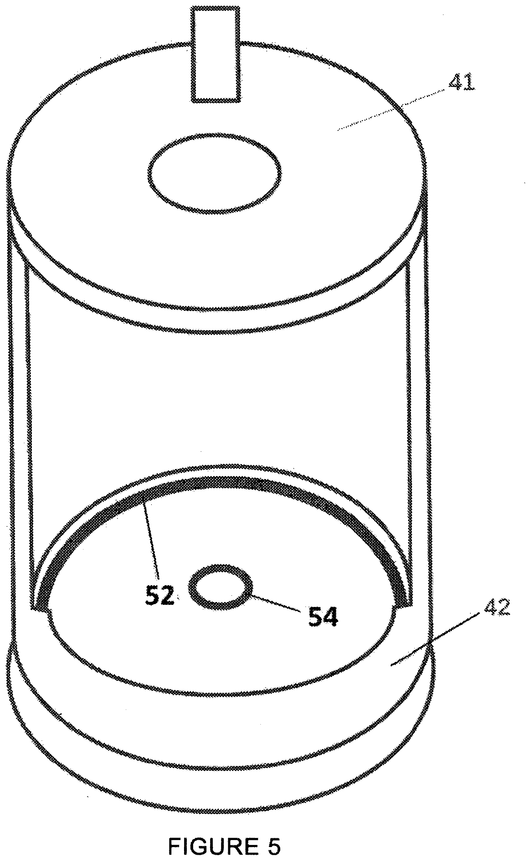

Figure 5: in this view similar to that portrayed in figure 4, wherein the doors (43, 44) have been eliminated, the disposition of a drain (54) for the egress of products in the body (42) of the cubicle (100) is seen.

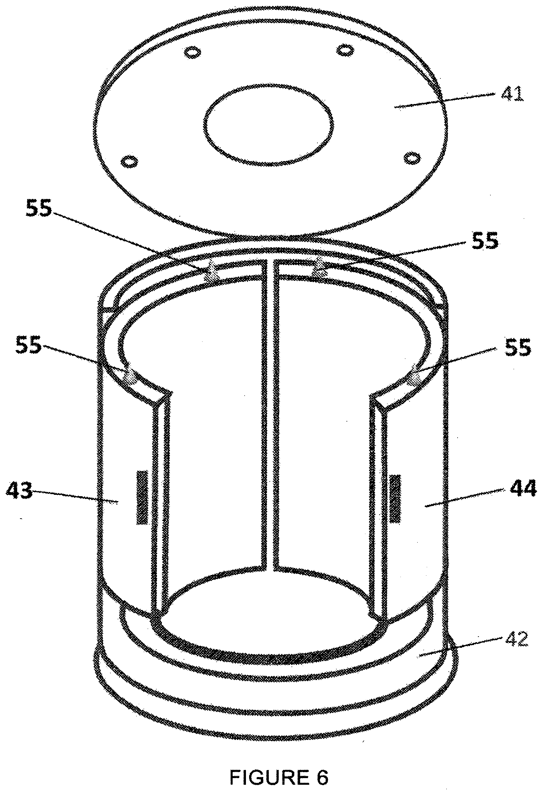

Figure 6: in this view similar to that portrayed in figure 3, wherein the seat (51) and the sprayers (49) have been eliminated, the disposition of a number of safety locks (55) disposed between the doors (43, 44) and the cover (41) may be seen.

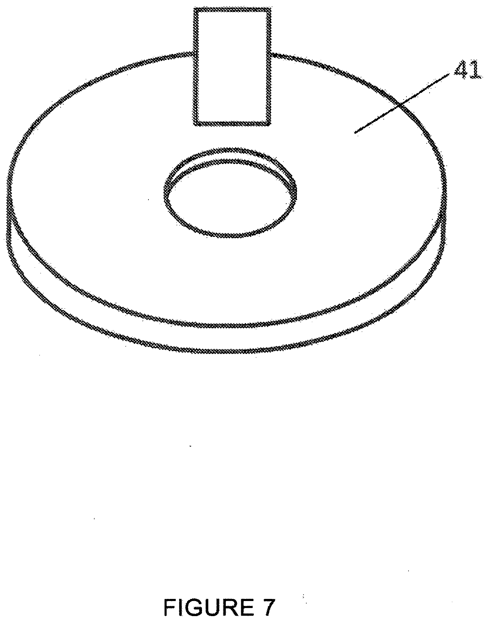

Figure 7: portrays a view of the cover (41) of the cubicle (100).

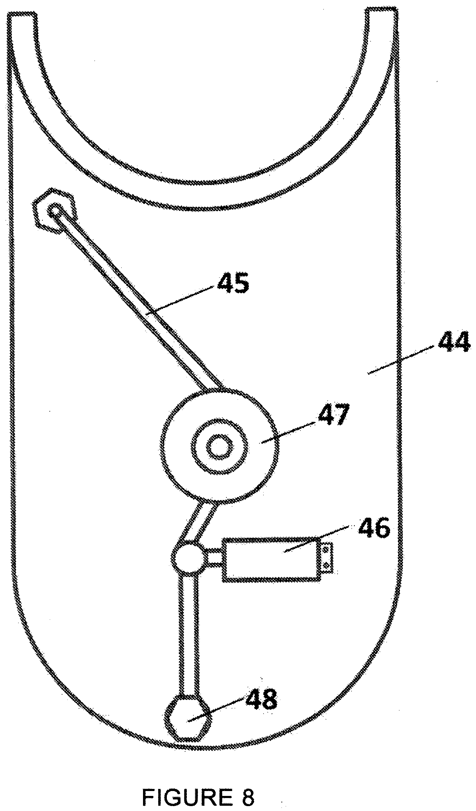

Figure 8: portrays a view of the interior of the right-hand door (44), where an elastic hose (45) of the corresponding outlet pipe is disposed, and features an inlet connection (48), the elastic hose (45) being held by a pulley (47) and a tensioner (46).

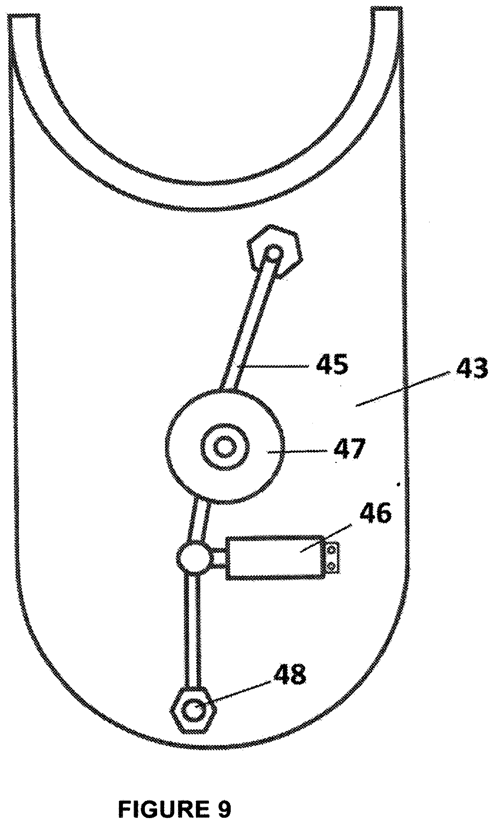

Figure 9: portrays a view of the interior of the left-hand door (43), where another elastic hose (45) of the corresponding outlet pipe is likewise disposed, featuring an inlet connection (48), and the elastic hose (45) being held by a pulley (47) and a tensioner (46).

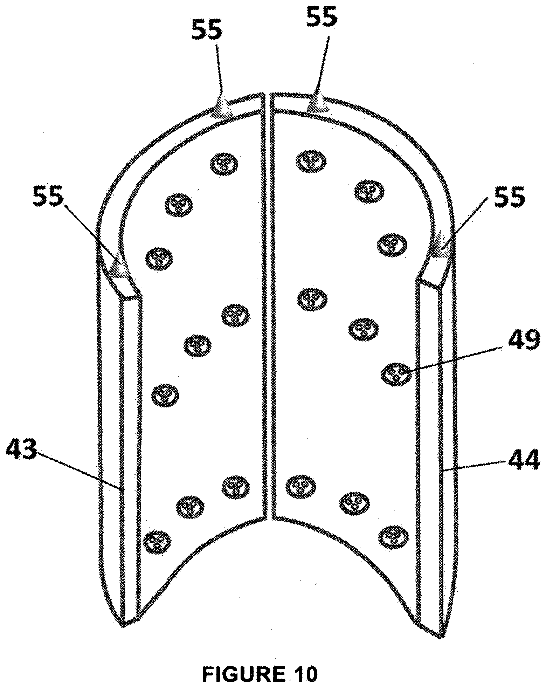

Figure 10: portrays a detail of the doors (43, 44) with the sprayers (49) and the safety locks (55).

### LIST OF REFERENCES EMPLOYED:

(1) Air pump (compressor) to provide compressed air.
(2) Non-return air valve disposed at the outlet of the air pump or compressor.
(3) First cistern (contains detergent, cosmetic and perfumed product for skin care, in accordance with the use of the cubicle).
(4) First pressure gauge (of the first cistern).
(5) First cover (of the first cistern).
(6) First safety valve (of the first cistern).
(7) First compressed air shutoff valve (to the first cistern).
(8) Detergent (contained in the first cistern).
(9) First flowmeter (of the first cistern).
(9a) First automatic valve which closes when the detergent is exhausted (of the first flowmeter of the first cistern).
(10) Flowmeter body (of the first flowmeter).
(11) First hinge (for the connection between the first cover and the first cistern, to enable the opening thereof).
(12) Holding tank for compressed air for the propulsion of the products employed during the operation of the cubicle.
(13) Compressed air supply duct (connected to the tank and to the product cisterns).
(14) Linking hose (between the duct (13) and the tank (12)).
(15)
(16) First pipe (for the connection between the first cistern and the programmer).
(17) Second pipe (for the connection between the second cistern and the programmer).
(18) Second cistern (for disinfectant).
(19) Second pressure gauge (of the second cistern (disinfectant)).
(20) Second cover (of the second cistern).
(21) Second hinge (for the connection between the second cover and the second cistern, to enable the opening thereof).
(22) Second safety valve (of the second cistern).
(23) Second compressed air shutoff valve (to the second cistern).
(24) Second flowmeter (of the second cistern).
(25) Second automatic valve which closes when the product contained is exhausted (of the second flowmeter of the second cistern).
(26)
(27) Third cistern (rinsing agent).
(28) Third pressure gauge (of the third cistern).
(29) Third cover (of the third cistern).
(30) Third hinge (for the connection between the third cover and the third cistern, to enable the opening thereof).
(31) Third safety valve (of the third cistern).
(32) Third compressed air shutoff valve (to the third cistern).
(33) Third flowmeter (of the third cistern).
(34) Third automatic valve which closes when the rinsing agent is exhausted (of the third flowmeter of the third cistern).
(35) Guides (of the automatic valves closing the cisterns).
(36) Programmer.
(37) Third pipe (connection between the third cistern and the programmer).
(38) Outlet pipe (from the programmer to the sprayers).
(39) Windows through which the outlet pipe (38) accesses the interior of the cubicle for its connection to the sprayers.
(40) Cubicle cover damper, facilitating the opening thereof.
(41) Cubicle cover.
(42) Cubicle body.
(43) Left-hand door.
(44) Right-hand door.
(45) Elastic hoses.
(46) Tensioner (to hold each elastic hose).
(47) Pulley (to hold each elastic hose).
(48) Inlet connectors (for the elastic hoses (45)).
(49) Sprayers.
(50)
(51) Seat (height-adjustable).
(52) Rails (on which the doors (43, 44) travel).
(53) Roller bearings (disposed in the doors (43, 44)).
(54) Cubicle drain.
(55) Safety locks disposed in the doors.
(100) Cubicle, as a whole.

The nature of the present invention having been sufficiently described, likewise the method for putting the same into practice, it must be stated that the dispositions indicated above and portrayed in the attached drawings are susceptible to modifications in detail provided that they do not alter the fundamental principle thereof.

## Claims

1. A disinfection cubicle (100) for personal hygiene and killing pathogenic bacteria and viruses, **characterised in that** it comprises a cylindrical body (42) having an upper cover (41) provided with a central hole for the hood of a biological protection overall against viruses and pathogenic bacteria worn by the user - or for the head of the user -, and a number of access doors (43, 44); the cubicle contains a number of cisterns (3, 18, 27) to carry products for disinfection and/or for the performance of personal hygiene processes; further comprising a tank (12) for the accumulation of compressed air for the propulsion of said products; pipes (16, 17, 37, 38) for carrying the products to a number of sprayers (49) disposed on the internal part of the doors (43, 44) of the cubicle (100), a programmer (36) interposed between the pipes (16, 17, 37) emerging from the cisterns (3, 18, 27) and the outlet pipe (38) connecting the programmer (36) to the sprayers (49) to regulate said propulsion and to perform the application of the products programmed; also comprising a compressed air pump (1) to accumulate air pressure within the tank (12); the cisterns comprise a first cistern (3) for detergent, cosmetic and perfumed products for skin care, a second cistern (18) for disinfectant and a third cistern (27) for rinsing agent, each of these having a cover (5, 20, 29), a flowmeter (9, 24, 33) for the product, a pressure gauge (4, 19, 28) for the measurement of pressure, and a safety valve (6, 22, 31); a height-adjustable seat (51) is located within the cubicle (100).

2. A disinfection cubicle (100) for personal hygiene and killing pathogenic bacteria and viruses, according to claim 1, **characterised in that** the doors (43, 44) are slidingly installed on a lower rail (52).

3. A disinfection cubicle (100) for personal hygiene and killing pathogenic bacteria and viruses, according to claim 1, **characterised in that** the first flowmeter (9) of the first cistern (3) comprises a first automatic valve (9a) which closes when the detergent is exhausted; while the first safety valve (6) is disposed in an air supply duct (13) to the first cistern (3) and is operated (closed) by the opening movement of a first cover (5) corresponding to said first cistern (3).

4. A disinfection cubicle (100) for personal hygiene and killing pathogenic bacteria and viruses, according to claim 1, **characterised in that** the second flowmeter (24) of the second cistern (18) of disinfectant comprises a second automatic valve (25) which closes when the product contained is exhausted; while the second safety valve (22) is disposed in an air supply duct (13) to the second cistern (18) and is operated (closed) by the opening movement of the second cover (20) corresponding to said second cistern (18).

5. A disinfection cubicle (100) for personal hygiene and killing pathogenic bacteria and viruses, according to claim 1, **characterised in that** the third flowmeter (33) of the third cistern (27) of rinsing agent comprises a third automatic valve (34) which closes when the rinsing agent is exhausted; while the third safety valve (31) is disposed in an air supply duct (13) to the third cistern (27) and is operated (closed) by the opening movement of the third cover (29) corresponding to said third cistern (27) .

6. A disinfection cubicle (100) for personal hygiene and killing pathogenic bacteria and viruses, according to claim 1, **characterised in that** the first pipe (16) is connected to the programmer (36) and to the first cistern (3) for the supply of detergent, cosmetic and perfumed product for skin care; the second pipe (17) is connected to the programmer (36) and to the second cistern (18) for the supply of disinfectant; and the third pipe (37) is connected to the programmer (36) and to the third cistern (27) for the supply of rinsing agent; while the outlet pipe (38) is connected to the sprayers (49) so that the product selected by the programmer (36) is released therefrom.

7. A disinfection cubicle (100) for personal hygiene and killing pathogenic bacteria and viruses, according to claim 1, **characterised in that** the cover (41) of the cubicle (100) comprises a damper (40) to facilitate its opening and closure.

8. A disinfection cubicle (100) for personal hygiene and killing pathogenic bacteria and viruses, according to claim 1, **characterised in that** the outlet pipes (38) comprise a number of elastic hoses (45) which are held by a pulley (47) and a tensioner (46).

9. A disinfection cubicle (100) for personal hygiene and killing pathogenic bacteria and viruses, according to claim 1, **characterised in that** it comprises a set of roller bearings (53) disposed in the doors (43, 44).

10. A disinfection cubicle (100) for personal hygiene and killing pathogenic bacteria and viruses, according to claim 1, **characterised in that** in the body (42) of the cubicle (100) it comprises a drain (54) for the egress of products.

11. A disinfection cubicle (100) for personal hygiene and killing pathogenic bacteria and viruses, according to any of the preceding claims, **characterised in that** it comprises a number of safety locks (55) disposed between the doors (43, 44) and the cover (41).
